# EUROPÄISCHE PATENTSCHRIFT

(11) **EP 0 596 484 B1**
(45) Veröffentlichungstag und Bekanntmachung des Hinweises auf die Patenterteilung: **19.03.1997**
(21) Anmeldenummer: 93117857.8
(22) Anmeldetag: 04.11.1993
(51) Int. Cl.: C07C 53/02, C07C 51/09

(54) **Ein Verfahren zur Herstellung von Ameisensäure**
Process for preparing formic acid
Procédé de préparation de l'acide formique

(30) Priorität: 05.11.1992 DE 4237339
(43) Veröffentlichungstag der Anmeldung: 11.05.1994
(73) Patentinhaber: Salzgitter Anlagenbau GmbH, 38239 Salzgitter (DE); BORISLAWER WISSENSCHAFTS- UND FORSCHUNGSINSTITUT BNII " SYNTHEZ", Borislaw 293 760 (UA)
(72) Erfinder: Sobotta, Georg, Dr.-Ing., D-38239 Salzgitter (DE); Pazderski, Juri Antonowitsch, Prof. Dr., UA-293760 Borislaw, Gebiet Lwow (UA); Skatschko, Wladimir Petrowitsch, Dr., UA-293760 Borislaw, Gebiet Lwow (UA); Tagajew, Oleg Alexejewitsch, Dr., UA-293760 Borislaw, Gebiet Lwow (UA)
(74) Vertreter: Lüdtke, Frank

(56) Entgegenhaltungen:
- DE-A- 3 220 555
- DE-B- 1 155 780
- FR-A- 1 602 512
- US-A- 2 719 166
- PATENT ABSTRACTS OF JAPAN vol. 9, no. 104 (C-279)(1827) 8. Mai 1985 & JP-A-59 231 027 (ORGANO K.K.) 25. Dezember 1984

## Beschreibung

Die Erfindung gehört zum Gebiet der organischen Chemie, konkret zu den Verfahren zur Herstellung von Ameisensäure, welche als Konservierungsmittel für Grünfutter für die Tierhaltung genutzt werden kann. Sie dient als Ausgangsstoff für die Synthese pharmazeutischer Präparate, für Dimethylformamid, Oxamid, Ammoniumformiat, für die Synthese von Fungiziden, Herbiziden, Insektiziden sowie für die Stabilisierung von Pestiziden, die als aktive Substanz Phosphate, Phosphonate und deren Derivate enthalten.

Bekannt ist ein Verfahren zur Herstellung von Ameisensäure mittels Hydrolyse von Methylformiat in Anwesenheit eines sauren Katalysators - schwach saure Kationenaustauscher - bei 55 bis 62 Grad Celsius und einem Verhältnis von Wasser zu Methylformiat von 14:1. Der Grad der Umwandlung von Methylformiat beträgt 87 Ma. - %. (M. J. Vysotzki, M. M. Ketzlach, A. I. Rotanowa "Journal der angewandten Chemie", 1967, Band 40, Nr. 11, S. 2440 bis 2442).

Die Unzulänglichkeit des bekannten Verfahrens besteht in der Erfordernis, in dem Prozeß große Wassermengen einzusetzen, was zu einem starken Energieverlust beim Abtrennen der Ameisensäure aus der Wasserlösung und zur Zirkulation großer Wassermengen in den Apparaten führt.

Entsprechend dem technischen Wesen und den erreichten Ergebnissen kommt dem erfindungsgemäßen, hier vorgestellten Verfahren zur Erzeugung von Ameisensäure die bekannte Hydrolyse von Methylformiat mit Wasser bei 60 bis 150 Grad Celsius, einem Druck von 3 bis 5 atm, einem Molverhältnis von Wasser zu Methylformiat (0,3 bis 3) : 1, im Beisein eines Katalysators, eines Produktes der Ameisensäure, sowie im Beisein von Zusätzen in Form von Methanol am nächsten. Der Prozeß wird in einem Reaktor mit Rührvorrichtung ausgeführt. (US-PS 3907884, Veröffentlichung 1975). In diesem Fall beträgt die Ausbeute von 1 Liter Reaktionsgemisch 79,0 Gramm/Stunde Ameisensäure.

Die Unzulänglichkeit des Verfahrens besteht darin, daß als Katalysator ein Reaktionsprodukt benutzt wird. Das führt zur Verringerung der Gleichgewichtsumwandlung von Methylformiat sowie der Produktivität des Reaktionsvolumens und zur Notwendigkeit, das Volumen der Apparaturen im Trennstadium zu vergrößern und ergibt damit auch Verluste in energetischer und finanzieller Hinsicht. Mit dem technologischen Schema, dargestellt in US-PS 390 7884, wird die Produktkonzentration der Ameisensäure (85 Ma. - %) aufgrund ihrer thermischen Instabilität nicht erreicht. (Masse % entspricht Gewichts %).

Bekannt ist ein Verfahren zur Herstellung von Ameisensäure (AMS) durch Hydrolyse von Methylformiat (MF) mit Wasser (W) bei einem Molverhältnis derselben von 1:1 bis 1:3 unter Erhitzen in Gegenwart eines Katalysators vom Säuretyp bei kontinuierlicher Betriebsweise. Die Hydrolyse erfolgt dabei in einem stehenden Zweizonen-Kolonnenreaktor bei Umgebungsdruck unter Einleiten des W in die obere Zone und des MF in die untere Zone im Gegenstrom des Reaktors. In der oberen Zone wird die Hydrolyse durch Wärmezufuhr bei konstanten 60 Grad Celsius oder bis 100 Grad Celsius durchgeführt, wobei ein Gemisch von nichtumgesetztem MF und gebildetem Methanol (ME) oben abgeleitet wird. In der unteren Zone des Reaktors erfolgt die Hydrolyse autokatalytisch in Gegenwart der aus der oberen Zone tretenden AMS bei einem pH-Wert < 1,9 und durch Wärmezufuhr bei konstanten 100 Grad Celsius oder bis 107 Grad Celsius unter gleichzeitigem Ableiten eines Gemisches von W, AMS und ME aus der unteren Zone, aus dem dann die AMS durch zweistufige Rektifikation gewonnen wird. Aus der oberen Zone des Reaktors werden 70 bis 99 % ME, bezogen auf die Gesamtmenge des sich bei der Reaktion gebildeten ME, und aus der unteren Zone 1 bis 30 % ME abgeleitet.

Die obere Zone des Reaktors ist mit einem stark sauren Sulfokationenaustauscher auf der Basis eines Kopolymerisates von Styrol und 2 bis 8 Gew.% Divinylbenzol als Katalysator gefüllt (DE-A-3 220 555).

Die Unzulänglichkeit des Verfahrens besteht darin, daß der eingesetzte Katalysator vor seiner Nutzung mit erhöhtem Aufwand aktiviert werden muß, was zu erheblichen zu entsorgenden Abwassermengen führt. Aufgrund der Zweiphasenströmung im Katalysatorbett und der erheblichen thermischen Belastung des Katalysators in der oberen Zone an der Reaktorwand durch Wärmezufuhr leidet die mechanische Festigkeit des Katalysators und führt zu einer Senkung seiner Betriebsfähigkeit. Weiterhin unvorteilhaft ist der bis zu 30 %ige Anteil des bei der Reaktion gebildeten Methanols im Gemisch aus Ameisensäure, Wasser und o. a. Methanol, das aus der unteren Zone des Reaktors abgeführt wird. Aus diesem Gemisch wird die Ameisensäure durch zweistufige Rektifikation abgetrennt. Bei diesem Vorgang erfolgt eine Rückreaktion von Ameisensäure mit Methanol wieder zu Wasser und Methylformiat, was die Produktivität des Prozesses bezogen auf die Ameisensäure wieder bis zu 30 % verringert.

Das Ziel der Erfindung besteht in der Erhöhung der Produktivität des Prozesses.

Das angestrebte Ziel erreicht man durch kontinuierliche Hydrolyse von Methylformiat in einem Verdrängungsreaktor in Anwesenheit eines stark sauren Sulfokationenaustauschers (Sulfonsäure-Kationenaustauscher) bei 80 bis 100 Grad Celsius und einem Molverhältnis Wasser : Methylformiat (1 bis 1.5) : 1 mit anschließender Rektifikation der Ameisensäure aus dem Hydrolysegemisch der wäßrigen Lösung der Ameisensäure (bei einem Unterdruck von 0,2 bis 0,5 bar) und einer zweistufigen Rektifikation der erhaltenen wäßrigen Lösung bei zwei verschiedenen Drücken.

Das Verfahren zur Herstellung von Ameisensäure nach der vorgeschlagenen Erfindung unterscheidet sich von bekannten Verfahren durch die Katalysatoren - stark saure Sulfokationenaustauscher - durch die Anwendung eines Verdrängungsreaktors, Rektifikation der Ameisensäure aus dem Hydrolysegemisch bei verringertem Druck der wäßrigen Lösung der Ameisensäure ohne jegliche Vorbehandlung, eine zweistufige Rektifikation der wäßrigen Lösung der Ameisensäure bis zur geforderten Konzentration von mindestens 86 Ma. - %. In dem empfohlenen Verfahren beträgt die Produktivität von 1 Liter Reaktionsgemisch 667 bis 800 Liter Ameisensäure/Stunde. Als Katalysator kommt ein Kationenaustauscher für heterogene Katalyse zur Anwendung. Einer seiner Vorzüge für die Nutzung als Katalysator besteht darin, daß er sich leicht von der Reaktionsmasse abtrennen läßt.

Dank dessen entfällt die Notwendigkeit des Auswaschens des Katalysators, welches in einer homogenen sauren Katalyse zur Bildung großer Abwassermengen führt.

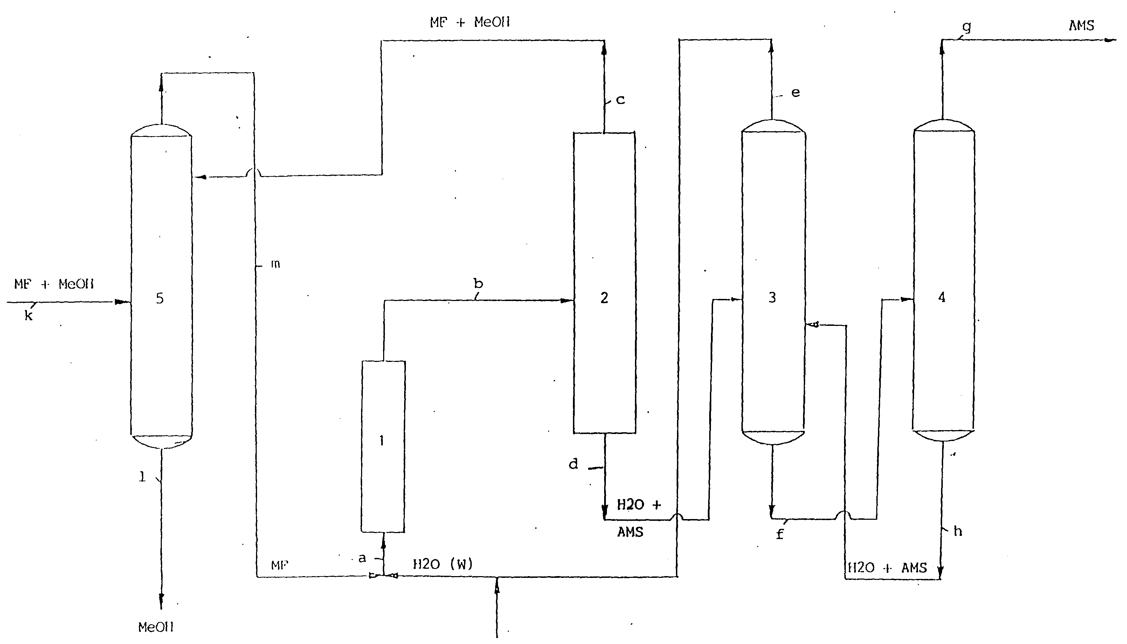

Der Vorteil des empfohlenen stark sauren Kationenaustauschers (H ⁺-Form) als Katalysator im Vergleich zu Mineralsäuren besteht in der größeren Selektivität bei der Umwandlung im Hydrolyseprozeß von Methylformiat zu Ameisensäure. Außerdem besitzt der Kationenaustauscher in H ⁺-Form eine große Aktivität bei der Hydrolyse vom Methylformiat, wodurch sich hohe Standzeiten im kontinuierlichen Prozeß ergeben. Das empfohlene Verfahren wird in einem Verdrängungsreaktor durchgeführt. Die Durchführung des Hydrolyseprozesses von Methylformiat in irgendeinen anderen Reaktor verschlechtert die Prozeßcharakteristika. Das Prinzipschema der Anlage, in der das empfohlene Verfahren durchgeführt wird, ist in der beigefügten Fig. 1 dargestellt. Im Prozess wird ein Verdrängungsreaktor 1 (rohr- oder tellerförmig) angewandt, dessen Teller oder Rohre mit dem Kationenaustauscher gefüllt werden. In solchen Apparaten erfolgt die Hydrolyse auf den Tellern oder in den Rohren, wo die Katalyse mit dem Kationenaustauscher stattfindet. Das sich bildende Reaktionsgemisch leitet man in die Rektifikationskolonne 2, die bei einem Druck von 0,2 bis 0,5 bar arbeitet. Das Sumpfprodukt dieser Kolonne - eine wäßrige Lösung der Ameisensäure - wird in einer zweistufigen Rektifikation in den Kolonnen 3 und 4 bis zu einer Konzentration von mindestens 86 Ma. - % angereichert. Das aus dem oberen Teil der Kolonne 2 austretende Gemisch von Methylformiat - Methanol wird in die Kolonne 5 zur Trennung geführt. Das abgetrennte Methylformiat geht zurück in den Reaktor 1 zur Hydrolyse.

### Beispiel 1 (erfindungsgemäßes Verfahren)

Die Anlage ist auf der beigefügten Figur dargestellt. Der Reaktor 1 für die Hydrolyse von Methylformiat besteht aus einem zylindrischen Apparat aus rostfreiem Stahl mit einem inneren Volumen von 3 dm³, gefüllt mit stark saurem großporigem Sulfokationenaustauscher in H ⁺-Form.

In den unteren Reaktorteil 1, erhitzt auf 90 Grad Celsius, werden 8,42 Kilogramm/Stunde Methylformiat (das 97,7 Ma. -% Methylformiat und 2,3 Ma. -% Methanol enthält) und 3,7 Kilogramm/Stunde Wasser gegeben (Teilstrom a). Das Molverhältnis Wasser zu Methylformiat beträgt 1,5 : 1. Das Produktgemisch bei Reaktionstemperatur (12,12 Kilogramm/Stunde), welches
43,40 Ma.- % Methylformiat (MF)
23,20 Ma.-.% Wasser (H₂O)
14,65 Ma.- % Methanol (MeOH)
18,75 Ma.- % Ameisensäure (AMS)
enthält (Teilstrom b), wird zur Trennung unmittelbar in die Rektifikationskolonne 2 geführt, in deren oberem Teil ein Druck von 0,5 bar besteht. Aus dem oberen Teil der Kolonne 2 wird bei T = 20 Grad Celsius kontinuierlich 7,21 Kilogramm/Stunde Alkohol-Ester-Gemisch zur Rektifikation abgeführt, wobei das Gemisch folgendes enthält (Teilstrom c):
22,05 Ma.- % Methanol
77,95 Ma.- % Methylformiat.

Aus dem Sumpf der Kolonne 2 werden bei 88 Grad Celsius kontinuierlich 4,91 Kilogramm/Stunde wäßrige Lösung der Ameisensäure, die 40,73 Ma. -% Säure enthält, abgeführt (Teilstrom d). Eine weitere Abtrennung der Ameisensäure erfolgt in zwei aufeinanderfolgenden Rektifikationskolonnen 3 und 4. In der Kolonne 3, in der im oberen Teil ein Überdruck von 1,8 bar herrscht, erhält man als Destillat 2,76 Kilogramm/Stunde Wasser, welches nach Vermischen mit dem Ausgangsstoff in den Reaktor 1 für die Hydrolyse von Methylformiat zurückgeführt wird (Teilstrom e). Das Sumpfprodukt (Teilstrom f) der Kolonne 3 (82 Ma. -%-.Konzentrat der Ameisensäure) gibt man in die Kolonne 4 zur Produktabtrennung, die mit einem Druck von 0,5 bar arbeitet. Aus dem oberen Teil der Kolonne 4 führt man 2,15 Kilogramm/Stunde 93,0 Ma. -% Ameisensäure bei 78 Grad Celsius ab (Teilstrom g). Das Sumpfprodukt der Kolonne 4, 72 % wäßrige Lösung der Ameisensäure, wird zum Entwässern in die Kolonne 3 zurückgeführt (Teilstrom h).

Im dargestellten Beispiel wird das Verfahren zur Herstellung von Ameisensäure bei einem anfänglichen Molverhältnis von Wasser und Methylformiat 1,5 : 1 und einer Temperatur von 90 Grad Celsius in Anwesenheit eines stark sauren Sulfokationenaustauschers in H ⁺-Form als Katalysator realisiert, wobei eine Produktivität von 666,7 Gramm/Stunde Ameisensäure bei 1 Liter Reaktionsvolumen erzielt wird.

### Beispiel 2 ( Vergleich mit Prototyp gemäß US-PS 39 07884)

In diesem Beispiel wird die Methode zur Herstellung von Ameisensäure entsprechend dem "Prototyp" experimentell überprüft, wobei vollständige Aussagen über die Produktivität nicht vorhanden sind.

In einen Reaktor (mit vollständiger Vermischung) mit einem Volumen von 20 dm³ werden 19,12 Kilogramm/Stunde Methylformiat, (welches 96,1 Ma. -% Methylformiat, 3,9 Ma. -% Methanol enthält), und 5,88 Kilogramm/Stunde Wasser, (welches 6,35 Ma. -% Ameisensäure enthält), gegeben. Die Zusammensetzung des Ausgangsreaktionsgemisches beträgt:
73,45 Ma.- % Methylformiat
22,05 Ma.- % Wasser
3,00 Ma.- % Methanol
1,50 Ma.- % Ameisensäure

Das Ausgangsverhältnis von Waser zu Methylformiat beträgt 1 : 1. Der Reaktor wird bei einer Temperatur von 80 Grad Celsius gehalten; mit einer Geschwindigkeit von 25,0 Kilogramm/Stunde wird ein Produktgemisch mit folgender Zusammensetzung abgeführt:
53,75 Ma.- % Methylformiat
16,15 Ma.- % Wasser
13,60 Ma.- % Methanol
16,15 Ma.- % Ameisensäure.

Die Produkte der Hydrolyse von Methylformiat werden zur Trennung in die Rektifikationskolonne abgeführt, in der Atmosphärendruck herrscht. Als Destillat wird aus dem oberen Kolonnenteil 18,38 Kilogramm/Stunde Alkohol-Ester-Gemisch gewonnen, welches 91,38 Ma.- % Methylformiat und 8,62 Ma.- % Methanol enthält, welches zur Abtrennung einzelner Komponenten weitergeleitet wird. Das Sumpfprodukt der Kolonne (6,62 Kilogramm/Stunde), welches 23,80 Ma.- % Ameisensäure und 76,20 Ma.- % Wasser enthält, wird einer Rektifikation zur Entwässerung unterworfen, wobei in der Kolonne ein Überdruck eingestellt wird, der aufgrund der thermischen Stabilität der Ameisensäure maximal möglich ist (2,05 bar).

Aus dem oberen Kolonnenteil für die Reinigung von Ameisensäure führt man 4,73 Kilogramm/Stunde Wasser ab, aus dem Sumpfprodukt erhält man 1,89 Kilogramm/Stunde 83,6 Ma.- %ige Ameisensäure. Die Versuche, ein Produkt mit 85 % Konzentration nach dem Schema, aufgezeigt im Patent US 39 07884, - Prototyp - (einstufige Rektifikation), bei geringer Erhöhung des Überdrucks in der Kolonne (bis 2,05 bar) zu erhalten, brachten nicht die gewünschten Resultate. Als maximale Konzentration der Ameisensäure wurden 83,6 Ma.- % bei einer Temperatur des Sumpfproduktes von 140 Grad Celsius erreicht.

Statt dessen führte die Druckerhöhung und dementsprechend die Temperatur des Sumpfproduktes zur Entcarbonylierung der Ameisensäure und damit zur Verringerung der Konzentration. In dem dargestellten Beispiel zur Realisierung des Verfahrens zur Herstellung von Ameisensäure entsprechend "Prototyp" wurde eine Produktivität von 79,00 Gramm/Stunde Ameisensäure je 1 Liter Reaktionsvolumen erzielt, wobei die Konzentration im abgenommenen Produkt nicht höher als 83,60 Ma.-% war.

Mit dem im Beispiel 1 dargestellten Verfahren wurde dagegen eine höhere Produktivität 666,70 Gramm/Stunde Ameisensäure je 1 Liter Reaktionsvolumen sowie eine höhere Produktkonzentration (93 Ma.- %) erreicht.

### Beispiel 3 bis 5 (siehe Tabelle)

Der Prozeß zur Herstellung von Ameisensäure wird in einer Anlage, dargestellt im Beispiel 1 und schematisch abgebildet in der Zeichnung, durchgeführt. In den Beispielen sind Grenzen für die Durchführbarkeit der vorgeschlagenen Methoden aufgezeigt, in denen das angestrebte Ziel erreicht wird, zum einen durch Veränderungen der Prozeßbedingungen, zum anderen durch Veränderungen der Parameter zur Trennung der Produkte.

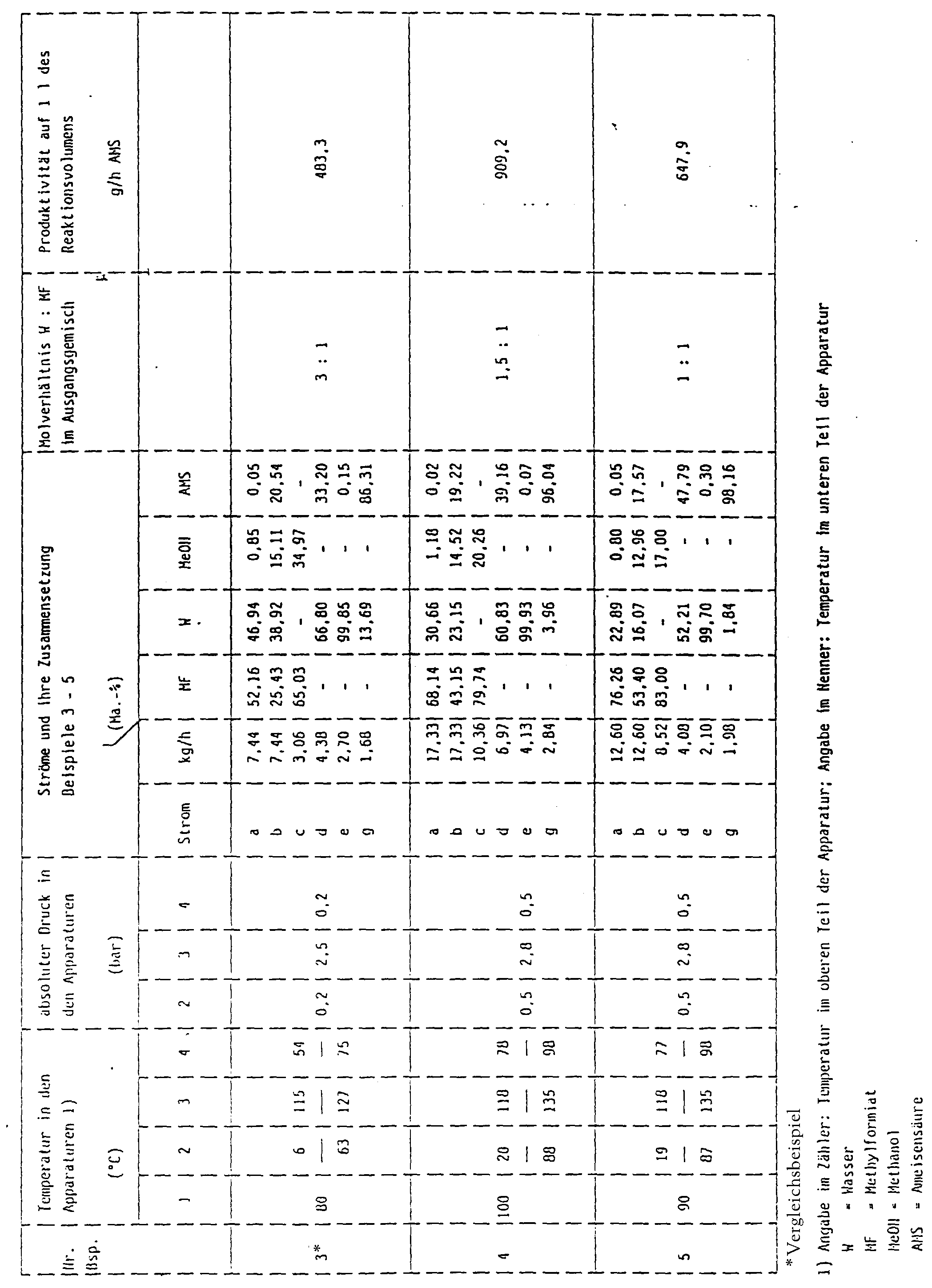

## Patentansprüche

1. Verfahren zur Herstellung von Ameisensäure durch Hydrolyse von Methylformiat mit Wasser bei Erwärmung bei einem Verhältnis Wasser Methylformiat von 1 bis 1,5 : 1 im Beisein eines stark sauren Katalysators, gekennzeichnet dadurch, daß mit dem Ziel der Erhöhung der Produktivität des Prozesses die Hydrolyse von Methylformiat kontinuierlich bei 80 bis 100 Grad Celsius in einem Verdrängungsreaktor bei einem Verhältnis Wasser : Methylformiat von 1 bis 1,5 : 1 durchgeführt wird, der mit einem stark sauren Sulfokationenaustauscher als heterogenem Katalysator gefüllt ist und das sich bildende Reaktionsgemisch zur Trennung in eine Rektifikationskolonne, die mit einem Unterdruck von 0,8 bis 0,5 bar arbeitet, weitergeleitet wird, wobei das Sumpfprodukt dieser Kolonne - wäßriges Produkt der Ameisensäure - einer zweistufigen Rektifikation bei zwei verschiedenen Drücken bis zum Erreichen einer Konzentration der Ameisensäure von mindestens 86 Ma.- % unterworfen wird.

## Claims

1. Method of producing formic acid by hydrolysing methyl formate with water whilst heating with a water : methyl formate ratio of between 1 and 1.5 : 1 in the presence of a strongly acidic catalyst, characterised in that, in order to increase the productivity of the process, the hydrolysis of methyl formate is continuously accomplished at between 80 and 100 degrees Celsius in a displacement reactor with a water : methyl formate ratio of between 1 and 1.5 : 1, which reactor is filled with a strongly acidic sulpho-cation exchanger as the heterogeneous catalyst, and the resultant reaction mixture is passed to a rectification column for separation purposes, which column operates at a reduced pressure of between 0.8 and 0.5 bar, the sump product of this column - aqueus product of formic acid - being subjected to a two-stage rectification at two different pressures until a formic acid concentration of at least 86 % by wt. is achieved.

## Revendications

1. Procédé de préparation de l'acide formique par hydrolyse du formiate de méthyle avec de l'eau et par chauffage, le rapport eau : formiate de méthyle étant de 1 à 1,5 , en présence d'un catalyseur acide fort, caractérisé en ce que, dans le but d'augmenter la productivité du procédé, l'hydrolyse du formiate de méthyle est menée en continu entre 80 et 100°C dans un réacteur de passage et avec un rapport eau : formiate de méthyle de 1 à 1,5, réacteur qui est rempli d'un échangeur à ions sulfocationiques en tant que catalyseur hétérogène, et que le mélange réactionnel se formant est mené pour séparation dans une colonne de rectification , opérant sous pression réduite de 0,8 à 0,5 bar, dans lequel le produit résiduel de cette colonne - qui est une composition aqueuse d'acide formique - est soumis à une rectification en deux étapes à deux pressions différentes jusqu'à obtenir une concentration massique en acide formique d'au moins 86 %.
